(19) Europäisches Patentamt
European Patent Office
Office européen des brevets

(11) Veröffentlichungsnummer: **0 306 875**
**A2**

(12) **EUROPÄISCHE PATENTANMELDUNG**

(21) Anmeldenummer: 88114432.3

(22) Anmeldetag: 03.09.88

(51) Int. Cl.4: C07D 307/32

(30) Priorität: 09.09.87 DE 3730186
02.02.88 DE 3802980

(43) Veröffentlichungstag der Anmeldung: 15.03.89 Patentblatt 89/11

(84) Benannte Vertragsstaaten: BE CH DE FR GB IT LI NL

(71) Anmelder: BASF Aktiengesellschaft
Carl-Bosch-Strasse 38
D-6700 Ludwigshafen(DE)

(72) Erfinder: Kummer, Rudolf, Dr.
Kreuzstrasse 6
D-6710 Frankenthal(DE)
Erfinder: Schneider, Heinz-Walter, Dr.
Bruesseler Ring 43
D-6700 Ludwigshafen(DE)
Erfinder: Bertleff, Werner, Dr.
Neuzenlache 3
D-6806 Viernheim(DE)
Erfinder: Fischer, Rolf, Dr.
Bergstrasse 98
D-6900 Heidelberg(DE)
Erfinder: Vagt, Uwe, Dr.
Paul-Neumann-Strasse 44
D-6720 Speyer(DE)
Erfinder: Naeumann, Fritz
Rathenaustrasse 3 a
D-6800 Mannheim 1(DE)
Erfinder: Hoelderich, Wolfgang, Dr.
Mannheimer Strasse 18 c
D-6710 Frankenthal(DE)
Erfinder: Schwarzmann, Matthias, Dr.
Carl-Bosch-Strasse 54
D-6703 Limburgerhof(DE)

(54) **Verfahren zur Herstellung von 5-Methylbutyrolacton.**

(57) Verfahren zur Herstellung von 5-Methylbutyrolacton, indem man Pentensäureester der Formel I

$X\text{-}CO_2R$ (I),

in der X die Reste $CH_2=CH\text{-}CH_2\text{-}CH_2\text{-}$, $CH_3\text{-}CH=CH\text{-}CH_2\text{-}$ oder $CH_3\text{-}CH_2\text{-}CH=CH\text{-}$ bedeutet und R für Alkylreste, Cycloalkylreste, Aralkyl- oder Arylreste steht, oder Gemische dieser Ester bei Temperaturen von 50 bis 350 °C mit Wasser gegebenenfalls in Gegenwart eines Verdünnungsmittels

a) an Zeolith- und/oder Phosphat-Katalysatoren umsetzt oder

b) in Gegenwart von 0,01 bis 0,25 Mol, pro Mol Pentensäureester, an Sulfonsäuren, Lewissäuren und/oder nicht-oxidierenden Mineralsäuren oder über 0,1 bis 40 Gew.%, bezogen auf den Pentensäureester, stark saurer Ionenaustauscher als Katalysatoren in einer Stufe umsetzt, oder daß man in einer ersten Stufe den Pentensäureester der Formel I, in der X und R die angegebenen Bedeutungen haben, mit Hilfe

EP 0 306 875 A2

eines stark sauren Ionenaustauschers als Katalysator zur Pentensäure der Formel I, in der R Wasserstoff bedeutet, hydrolysiert und die so erhaltene Pentensäure in einer zweiten Stufe in Gegenwart von 0,005 bis 0,1 Mol, pro Mol Pentensäure, an Sulfonsäuren, Lewissäuren oder nicht-oxidierenden Mineralsäuren oder über 0,1 bis 20 Gew.%, bezogen auf die Pentensäure, eines stark sauren Ionenaustauschers bei Temperaturen von 50 bis 350 °C cyclisiert.

## Verfahren zur Herstellung von 5-Methylbutyrolacton

Die Erfindung betrifft ein Verfahren zur Herstellung von 5-Methylbutyrolacton durch Umsetzung von Pentensäureestern mit Wasser in Gegenwart von sauren Katalysatoren.

Aus US-PS 2 509 859 und DE 896 343 ist bekannt, daß 5-Methylbutyrolacton durch Cyclisierung von Pentennitrilen mit nichtoxidierenden Mineralsäuren, wie Salzsäure oder Schwefelsäure entsteht. Diese Methode hat jedoch den Nachteil, daß ein Zwangsanfall an equimolaren Mengen Ammoniumsalzen auftritt.

Darüber hinaus kann 5-Methylbutyrolacton nach einer ganzen Reihe von Veröffentlichungen auch durch Reduktion von Lävulinsäure bzw. deren Estern (z.B. mit Natriumamalgam, komplexen Hydriden, wie Lithiumborhydrid, oder Wasserstoff in Gegenwart von geeigneten Hydrierkatalysatoren) hergestellt werden. So wird beispielsweise in EP 069 409 die Hydrierung von Lävulinsäureestern mit Wasserstoff an Ni/Co-Katalysatoren beschrieben.

Weiterhin konnte 5-Methylbutyrolacton gemäß US-PS 2 420 250 durch eine unter Dehydrierung verlaufende Cyclisierung von 1,4-Pentandiol an Kupfer/Chromit-Katalysatoren oder gemäß DE-OS 21 22 501 durch Isomerisierung von δ-Valerolacton an Borsäure/Phosphorsäure-Katalysatoren hergestellt werden.

Die genannten Methoden sind für ein technisches Verfahren weniger gut geeignet, da die Ausgangsprodukte zum einen teuer und zum anderen auch nicht in entsprechend großen Mengen verfügbar sind.

Es war ferner bekannt, Pentensäuren mit Schwefelsäure zu 5-Methylbutyrolacton zu cyclisieren. So wird beispielsweise in J. Chem. Soc. 1963, Seiten 2640 bis 2644 die Cyclisierung von 4-, 3-trans- und 2-trans-Pentensäure mit 50 %iger Schwefelsäure beschrieben. Die dabei erhaltenen Ausbeuten an 5-Methylbutyrolacton sind mit knapp 50 % allerdings sehr unbefriedigend. Zudem wird gemäß dieser und auch bei allen früheren Arbeiten (wie Liebigs Annalen 283 (1894), Seite 86 und J. Chem. Soc. 1933, Seiten 577 bis 586) mit unwirtschaftlich hohen Schwefelsäuremengen (ca. 10 g Schwefelsäure pro g Pentensäure) gearbeitet. Dieser Umstand hat außerdem noch ein sehr aufwendiges Verfahren zur Isolierung des Lactons, z.B. durch Wasserdampfdestillation aus den Reaktionsgemischen zur Folge.

Es bestand daher die Aufgabe, Verfahren vorzuschlagen, die von gut zugänglichen Ausgangsmaterialien ausgehen und in guten Ausbeuten 5-Methylbutyrolacton liefern.

Diese Aufgabe wurde erfindungsgemäß mit einem Verfahren zur Herstellung von 5-Methylbutyrolacton gelöst, gemäß dem man Pentensäureester der Formel

$X-CO_2R$ (I)

in der X die Reste $CH_2=CH-CH_2-CH_2-$,
$CH_3-CH=CH-CH_2-$ oder
$CH_3-CH_2-CH=CH-$ bedeutet
und R für Alkylreste, Cycloalkylreste, Aralkyl- oder Arylreste steht, oder Gemische dieser Ester bei Temperaturen von 50 bis 350 °C mit Wasser gegebenenfalls in Gegenwart eines Verdünnungsmittels

a) an Zeolith- und/oder Phosphat-Katalysatoren umsetzt oder

b) in Gegenwart von 0,01 bis 0,25 Mol, pro Mol Pentensäureester, an Sulfonsäuren, Lewissäuren und/oder nicht-oxidierenden Mineralsäuren oder über 0,1 bis 40 Gew.%, bezogen auf den Pentensäureester, stark saurer Ionenaustauscher als Katalysatoren in einer Stufe umsetzt, oder daß man in einer ersten Stufe den Pentensäureester der Formel I, in der X und R die angegebenen Bedeutungen haben, mit Hilfe eines stark sauren Ionenaustauschers als Katalysator zur Pentensäure der Formel I, in der R Wasserstoff bedeutet, hydrolysiert und die so erhaltene Pentensäure in einer zweiten Stufe in Gegenwart von 0,001 bis 0,1 Mol, pro Mol Pentensäure, an Sulfonsäuren, Lewissäuren oder nicht-oxidierenden Mineralsäuren oder über 0,1 bis 20 Gew.%, bezogen auf die Pentensäure, eines stark sauren Ionenaustauschers bei Temperaturen von 50 bis 350 °C cyclisiert.

Die erfindungsgemäße Reaktion läßt sich z.B. für die Umsetzung von 2-trans-Pentensäuremethylester zu 5-Methylbutyrolacton durch die folgende Reaktionsgleichung darstellen:

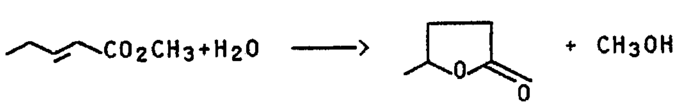

Die als Ausgangsstoff benötigten Ester der Formel I sind vor allem $C_1$- bis $C_{12}$-Alkyl-, $C_5$- bis $C_{10}$-Cycloalkyl-, insbesondere $C_5$- und $C_6$-Cycloalkyl, $C_7$- bis $C_{15}$-Aralkyl- oder $C_6$- bis $C_{10}$-Arylester der entsprechenden Säuren, wie 4-Pentensäure, 3-cis- oder 3-trans-Pentensäure, 2-trans- oder 2-cis-Pentensäure. Beispielhaft seien folgende Reste R genannt: Methyl-, Ethyl-, Propyl-, Isopropyl-, n-Butyl-, Isobutyl-, Sec.-Butyl-, tert.-Butyl-,Hexyl-, Nonyl-, Dodecyl-, Cyclopentyl-, Cyclohexyl-, Phenyl- oder Benzylreste, die gegebenenfalls in der Alkoholkomponente weiter substituiert werden können z.B. durch $C_1$- bis $C_4$-Alkyl, $C_1$-$C_4$-Alkoxy der Halogen wie Fluor, Chlor, Brom oder Jod. Besonders bevorzugte Ausgangsstoffe der Formel I sind Pentensäure-$C_1$- bis $C_6$-Alkylester.

Beispielsweise kommen folgende Pentensäureester der Formel I oder deren Gemische als Ausgangsstoffe in Betracht:
4-Pentensäuremethylester, 3-trans-Pentensäuremethylester, 3-cis-Pentensäuremethylester, 2-trans-Pentensäuremethyleser, 2-cis-Pentensäuremethylester, 4-Pentensäureethylester, 3-trans-Pentensäureethylester, 3-cis-Pentensäureethylester-, 2-trans-Pentensäureethylester, 2-cis-Pentensäureethylester, 4-Pentensäurepropylester, 3-trans-Pentensäurepropylester, 3-cis-Pentensäurepropylester, 2-trans-Pentensäurepropylester, 2-cis-Pentensäurepropylester, 4-Pentensäurebutylester, 3-trans-Pentensäurebutylester, 3-cis-Pentensäurebutyleser, 2-trans-Pentensäurebutylester, 2-cis-Pentensäurebutylester, 4-Pentensäurehexylester, 3-trans-Pentensäurenonylester, 3-cis-Pentensäuredodecylester, 2-trans-Pentensäurecyclopentylester, 2-cis-Pentensäurecyclohexylester, 4-Pentensäurebenzylester, 3-trans-Pentensäurebenzylester, 3-cis-Pentensäurephenylester, 2-trans-Pentensäurephenylester oder 2-cis-Pentensäurephenylester.

Die Umsetzung findet bei Temperaturen von 50°C bis 350°C, vorzugsweise bei 80 bis 260°C statt. Im allgemeinen wird die Reaktion unter Atmosphärendruck durchgeführt. Es ist jedoch auch möglich, schwach verminderten oder erhöhten Druck, von 0,01 bis zu 50 bar, anzuwenden.

Die Umsetzung wird in Gegenwart von Wasser durchgeführt. Im allgemeinen wendet man je Mol Pentensäureester der Formel I 0,2 bis 30, insbesondere 0,5 bis 20 mol, besonders bevorzugt 1 bis 10 mol Wasser an.

Als Katalysator für das erfindungsgemäße Verfahren verwendet man a) Zeolithe und/oder Phosphate.

Zeolithe sind kristalline Aluminiumsilikate, die eine hochgeordnete Struktur mit einem starren dreidimensionalen Netzwerk von $SiO_4$- und $AlO_4$-Tetraedern besitzen, die durch gemeinsame Sauerstoffatome verbunden sind. Das Verhältnis der Si- und Al-Atome zu Sauerstoff beträgt 1:2. Die Elektrovalenz der Aluminium enthaltenden Tetraeder ist durch Einfluß von Kationen in den Kristall, z.B. eines Alkali- oder Wasserstoffions ausgeglichen. Ein Kationenaustausch ist möglich. Die Räume zwischen den Tetraedern sind vor der Dehydration durch Trocknen bzw. Calcinieren von Wassermolekülen besetzt.

In den Zeolithen können anstelle von Aluminium auch andere Elemente wie B, Ga, Fe, Cr, V, As, Sb, Bi oder Be oder deren Gemische in das Gitter eingebaut werden, oder das Silicium kann durch ein vierwertiges Element wie Ge, Ti, Zr, Hf ersetzt werden.

Entsprechend ihrer Struktur werden Zeolithe in verschiedene Gruppen unterteilt. So bilden bei der Mordenit-Gruppe Ketten oder bei der Chabasit-Gruppe Schichten aus Tetraedern die Zeolith-Struktur, während sich bei der Faujasit-Gruppe die Tetraeder zu Polyedern ordnen, z.B. in Form eines Kubooktaeders, der aus Vierringen bzw. Sechsringen aufgebaut ist. Je nach Verknüpfung der Kubooktaeder, wodurch unterschiedlich große Hohlräume und Poren entstehen, unterscheidet man Zeolithe vom Typ A, L, X oder Y.

Für das erfindungsgemäße Verfahren in Betracht kommende Katalysatoren sind Zeolithe aus der Mordenit-Gruppe oder engporige Zeolithe vom Erionit- bzw. Chabasit-Typ oder Zeolithe vom Faujasit-Typ, z.B. Y-, X- oder L-Zeolithe. In diese Gruppe von Zeolithen gehören auch die sogenannten "ultrastabilen" Zeolithe des Faujasittyps, d.h. dealuminierte Zeolithe. Verfahren zur Herstellung solcher Zeolithe sind z.B. beschrieben in "Catalysis by Zeolites", Band 5 aus Studies in Surface Science and Catalysis" ed. B. Imelik et al. Elsevier Scientific Publishing Comp., 1980, S. 203 und "Crystal Structures of Ultra-stable Faujasites" Advances in Chemistry Series Nr. 101, American Chemical Society, Washington, DC, S. 226ff (1971) und in US-PS 4 512 961.

Besonders vorteilhaft sind Zeolithe vom Pentasiltyp. Diese haben als Grundbaustein einen aus $SiO_4$-Tetraedern aufgebauten Fünfring gemeinsam. Sie sind durch ein hohes $SiO_2/Al_2O_3$-Verhältnis gekennzeichnet sowie durch Porengrößen, die zwischen denen der Zeolithe vom Typ A und denen vom Typ X oder Y liegen (vgl. Ullmanns Encyclopädie d. techn. Chem., 4. Aufl., Bd. 24, 1983).

Diese Zeolithe können unterschiedliche chemische Zusammensetzung aufweisen. Es handelt sich hierbei um Alumino-, Boro-, Eisen-, Beryllium-, Gallium-, Chrom-, Arsen-, Antimon- und Wismutsilikatzeolithe oder deren Gemische sowie Alumino-, Boro-, Gallium- und Eisengermanatzeolithe oder deren Gemische. Insbesondere eignen sich die Alumino-, Boro- und Eisensilikatzeolithe des Pentasiltyps für das erfindungsgemäße Verfahren.

Der Aluminosilikatzeolith wird z.B. aus einer Aluminiumverbindung, vorzugsweise $Al(OH)_3$ oder $Al_2(SO_4)_3$ und einer Siliciumkomponente, vorzugsweise hochdispersem Siliciumdioxid in wäßriger Aminlösung, insbesondere in Polyaminen wie 1,6-Hexandiamin- oder 1,3-Propandiamin- oder Triethylentetramin-Lösung mit oder insbesondere ohne Alkali- oder Erdalkalizusatz bei 100 bis 220°C unter autogenem Druck

hergestellt. Auch gehören hierzu die isotaktischen Zeolithe nach EP 34 727 und EP 46 504. Die erhaltenen Aluminosilikatzeolithe enthalten je nach Wahl der Einsatzstoffmengen ein $SiO_2/Al_2O_3$-Verhältnis von 10 bis 40.000. Auch lassen sich derartige Aluminosilikatzeolithe in etherischem Medium wie Diethylenglykoldimethylether, in alkoholischem Medium wie Methanol bzw. 1,4-Butandiol oder in Wasser synthetisieren.

Der Borosilikatzeolith wird z.B. bei 90 bis 200 °C unter autogenem Druck synthetisiert, indem man eine Borverbindung z.B. $H_3BO_3$, mit einer Siliciumverbindung, vorzugsweise hochdispersem Siliciumdioxid in wäßriger Aminlösung, insbesondere in 1,6-Hexandiamin- oder 1,3-Propandiamin- oder Triethylentetramin-Lösung mit und insbesondere ohne Alkali- oder Erdalkalizusatz zur Reaktion bringt. Auch gehören hierzu die isotaktischen Zeolithe nach EP 34 727 und EP 46 504. Solche Borosilikatzeolithe können ebenfalls hergestellt werden, wenn man die Reaktion statt in wäßriger Aminlösung in etherischer Lösung, z.B. Diethylenglykoldimethylether oder in alkoholischer Lösung, z.B. 1,6-Hexandiol durchführt.

Den Eisensilikatzeolith erhält man z.B. aus einer Eisenverbindung, vorzugsweise $Fe_2(SO_4)_3$ und einer Siliciumverbindung, vorzugsweise hochdispersem Siliciumdioxid, in wäßriger Aminlösung, insbesondere 1,6-Hexandiamin, mit oder ohne Alkali- oder Erdalkalizusatz bei 100 bis 220 °C unter autogenem Druck.

Die so hergestellten Alumino-, Boro- und Eisensilikatzeolithe können nach ihrer Isolierung, Trocknung bei 100 bis 160 °C, vorzugsweise 110 °C und Calcinierung bei 450 bis 550 °C, vorzugsweise 500 °C, mit einem Bindemittel im Verhältnis 90:10 bis 40:60 Gew.% zu Strängen oder Tabletten verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$, Gemische aus hochdispersem $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Man erhält auch vorteilhafte Katalysatoren, wenn der isolierte Alumino-bzw. Borosilikatzeolith direkt nach der Trocknung verformt wird und erst nach der Verformung einer Calcinierung unterworfen wird. Die hergestellten Alumino- ud Borosilikatzeolithe können in reiner Form, ohne Binder, als Stränge oder Tabletten eingesetzt werden, wobei als Verstrangungs- oder Peptisierungshilfsmittel z.B. Ethylcellulose, Stearinsäure, Kartoffelstärke, Ameisensäure, Oxalsäure, Essigsäure, Salpetersäure, Ammoniak, Amine, Silikoester und Graphit oder deren Gemische verwendet werden.

Zu den erfindungsgemäß verwendbaren siliciumreichen Zeolithen ($SiO_2/Al_2O_3 \geq 10$) gehören auch die verschiedenen ZSM-Typen: ZSM-5 (US 3 702 886, US 3 832 449, US 4 076 859), Ferrierit (EP 12 473), Nu-1 (US 4 060 590) und Silicalit ® (US-PS 4 061 724).

Silicatite® lassen sich z.B. aus Kieselsolen in Gegenwart von Tetrapropylammoniumhydroxid und gegebenenfalls von Alkalihydroxid unter hydrothermalen Bedingungen bei Temperaturen von 150 bis 250 °C herstellen. Die so hergestellten pulverförmigen Silicalite® können nach ihrer Isolierung, Trocknung bei 100 bis 160 °C, vorzugsweise 110 °C und Calcinierung bei 350 bis 550 °C, vorzugsweise 450 bis 500 °C, mit einem Bindemittel im Verhältnis 90:10 bis 20:80 Gew.% zu Strängen oder Tabletten oder Wirbelgut verformt werden. Als Bindemittel eignen sich diverse Aluminiumoxide, bevorzugt Boehmit, amorphe Aluminosilikate mit einem $SiO_2/Al_2O_3$-Verhältnis von 25:75 bis 90:5, bevorzugt 75:25, Siliciumdioxid, bevorzugt hochdisperses $SiO_2$ und hochdispersem $Al_2O_3$, $TiO_2$, $ZrO_2$ sowie Ton. Nach der Verformung werden die Extrudate oder Preßlinge bei 110 °C/16 h getrocknet und bei 500 °C/16 h calciniert.

Liegt der Zeolith aufgrund der Art seiner Herstellung z.B. in der Na-Form vor, dann kann diese durch Ionenaustausch, z.B. mit Ammoniumionen und anschließende Calcinierung oder durch Behandlung mit Säuren vollkommen oder partiell in die H-Form überführt werden.

Weiterhin kann es vorteilhaft sein, die Zeolithe zu modifizieren. Eine geeignete Modifizierung der Katalysatoren besteht z.B. darin, daß man den unverformten oder verformten Zeolithen mit Metallsalzen durch einen Ionenaustausch oder durch Imprägnierung dotiert. Als Metalle können Alkalimetalle wie Li, Cs, K, Erdalkalimetalle wie Mg, Ca, Sr, Ba, Metalle der 3., 4. und 5. Hauptgruppe wie B, Al, Ga, Ge, Sn, Pb, Bi, oder Übergangsmetalle eingesetzt werden.

Zweckmäßigerweise führt man diese Dotierung so durch, daß man den verformten Zeolithen in einem Steigrohr vorlegt und bei 20 bis 100 °C eine wäßrige oder ammoniakalische Lösung eines Halogenids oder eines Nitrats der voranbeschriebenen Metalle überleitet. Ein derartiger Ionenaustausch kann an der Wasserstoff-, Ammonium- und Alkaliform des Zeolithen vorgenommen werden. Eine weitere Möglichkeit der Metallaufbringung auf den Zeolithen ist gegeben, indem man das zeolithische Material, z.B. mit einem Halogenid, einem Nitrat oder einem Oxid der voranbeschriebenen Metalle in wäßriger, alkoholischer oder ammoniakalischer Lösung imprägniert. Sowohl an einen Ionenaustausch als auch an eine Imprägnierung schließt sich zumindest eine Trocknung, wahlweise eine abermalige Calcinierung an.

Ein Ionenaustausch des in der H-Form oder Ammonium-Form oder Alkali-Form vorliegenden Zeolithen kann so vorgenommen werden, daß man den Zeolithen in Strängen oder Pellets in einer Kolonne vorlegt und darüber eine Lösung des Metallsalzes bei leicht erhöhter Temperatur zwischen 30 und 80 °C im

Kreislauf 15 bis 20 h leitet. Danach wird mit Wasser ausgewaschen, bei etwa 150°C getrocknet und bei etwa 550°C calciniert.

Weitere Katalysatoren für das erfindungsgemäße Verfahren sind Phosphate, insbesondere Aluminiumphosphate, Siliciumaluminiumphosphate, Boraluminiumphosphate, Siliciumeisenaluminiumphosphate, Cerphosphate, Zirkonphosphate, Borphosphate, Eisenphosphate, Strontiumphosphat oder deren Gemische.

Als Aluminiumphosphat-Katalysatoren werden für das erfindungsgemäße Verfahren insbesondere unter hydrothermalen Bedingungen synthetisierte Aluminiumphosphate eingesetzt.

Die unter hydrothermalen Bedingungen hergestellten Aluminiumphosphate (APO) sind z.B. APO-5, APO-9, APO-11, APO-12, APO-14, APO-21, APO-25, APO-31 und APO-33. Synthesen dieser Verbindungen sind in der EP 132 708; US 4 310 440 und US 4 473 663 oder von S.T. Wilson, B.M. Lok, C.A. Messina und E.M. Flanigen in Proc. 6th Fut. Zeolite Conf., Reno 1983, Herausgeber D.M. Olson, A. Bisio, Seiten 97 - 109 beschrieben.

Beispielsweise wird das $AlPO_4$-5 (APO-5) synthetisiert, indem man Orthophosphorsäure mit Pseudoboehmit (Catapal SB®) in Wasser homogen mischt; zu dieser Mischung Tetrapropylammoniumhydroxid gibt und danach bei ca. 150°C 20 bis 60 h unter autogenem Druck in einem Autoklaven umsetzt. Das abfiltrierte $AlPO_4$ wird getrocknet bei 100 bis 160°C und calciniert bei 450 bis 550°C.

$AlPO_4$-9 (APO-9) wird ebenfalls aus Orthophosphorsäure und Pseudoboehmit aber in wäßriger 1,4-Diazabicyclo-(2,2,2)octan-Lösung (DABCO-Lösung) bei ca. 200°C unter autogenem Druck während 200 bis 400 h synthetisiert. Verwendet man anstelle von DABCO-Lösung Ethylendiamin, so gelangt man zu APO-12.

Die Synthese des $AlPO_4$-21 (APO-21) erfolgt aus Orthophosphorsäure und Pseudoboehmit in wäßriger Pyrrolidon-Lösung bei 150 bis 200°C unter autogenem Druck während 50 bis 200 h.

Die für das erfindungsgemäße Verfahrend verwendbaren Siliciumaluminiumphosphate (SAPO) sind z.B. SAPO-5, SAPO-11, SAPO-31 und SAPO-34. Die Synthese dieser Verbindungen wird z.B. in EP 103 117 oder US-PS 4 440 871 oder von E.M. Flanigen et al in Pure Appl. Chem. 58, 1351-58 (1986) beschrieben. Siliciumaluminiumphosphate werden hergestellt durch Kristallisation aus wäßriger Mischung bei 100 bis 250°C und autogenem Druck während 2 h bis 2 Wochen, wobei die Reaktionsmischung aus einer Silicium-, Aluminium- und Phosphorkomponente in wäßrigen aminoorganischen Lösungen umgesetzt wird.

SAPO-5 beispielsweise wird durch Mischen von $SiO_2$ suspendiert in wäßriger Tetrapropylammoniumhydroxid-Lösung mit einer wäßrigen Suspension aus Pseudoboehmit und Orthophosphorsäure und anschließende Umsetzung bei 150 bis 200°C während 20 bis 200 h unter autogenem Druck in einem Rührautoklaven erhalten. Die Trocknung des abfiltrierten Pulvers erfolgt bei 110 bis 160°C und die Calcination bei 450 bis 550°C.

Als Siliciumaluminiumphosphate sind auch ZYT-5, ZYT-6, ZYT-7, ZYT-9, ZYTI-11 und ZYT-12 geeignet. Solche Siliciumaluminiumphosphate werden beispielsweise in der Japanischen Patentanmeldung 59217-619/1984 beschrieben.

Borphosphate für das erfindungsgemäße Verfahren lassen sich beispielsweise durch Mischung und Kneten von konzentrierter Borsäure und Phosphorsäure und durch anschließende Trocknung und Calcination in Inertgas-, Luft- oder Dampfatmosphäre bei 250 bis 650°C, vorzugsweise 300 bis 500°C, herstellen.

Als Phosphat-Katalysatoren kann man bei dem Verfahren auch gefällte Aluminiumphosphate einsetzen. Beispielsweise wird ein derartiges Aluminiumphosphat hergestellt, indem 92 g Diammoniumhydrogenphosphat in 700 ml Wasser gelöst werden. Zu dieser Lösung wird 260 g $Al(NO_3)_3 \times H_2O$ in 700 ml Wasser über 2 h zugetropft. Dabei wird der pH-Wert durch gleichzeitige Zugabe von 25 %iger $NH_3$-Lösung bei pH 8 gehalten. Der entstandene Niederschlag wird 12 h nachgerührt, dann abgesaugt und ausgewaschen. Er wird bei 60°C/16 h getrocknet.

Auf diese Phosphate können durch Imprägnierung (Tränken und Aufsprühen) oder in manchen Fällen auch durch Ionenaustausch Modifizierungskomponenten wie voran bei den Zeolithen beschrieben aufgebracht werden. Auch kann wie bei den Zeolithkatalysatoren eine Modifizierung mit Säuren, z.B. Phosphorsäure erfolgen.

Die hier beschriebenen Katalysatoren können wahlweise als 2- bis 4-mm-Stränge oder als Tabletten mit 3 bis 5 mm Durchmesser oder als Splitt mit Teilchengrößen von 0,1 bis 0,5 mm oder als Wirbelkontakt eingesetzt werden.

Die erfindungsgemäß zur Herstellung von 5-Methylbutyrolacton eingesetzten Katalysatoren zeigen auch über einen längeren Zeitraum, insbesondere beim Arbeiten in Alkohol keine Aktivitätsabnahme. Wenn bei der erfindungsgemäßen Verwendung der zeolithischen Katalysatoren wie auch der anderen hierfür eingesetzten Katalysatoren eventuell eine durch Koksabscheidung bedingte Desaktivierung eintritt, empfiehlt es sich, diese durch Abbrennen der Koksablagerung mit Luft oder mit einem Luft/$N_2$-Gemisch bei 400 bis 550°C, bevorzugt 500°C, zu regenerieren. Die Katalysatoren erhalten dadurch ihre Anfangsaktivität zurück.

Durch partielle Verkokung (pre-coke) ist es möglich, die Aktivität des Katalysators für ein Selektivitätsoptimum des gewünschten Reaktionsproduktes einzustellen.

Die Katalysatorbelastung liegt im allgemeinen bei 0,01 bis 40, vorzugsweise 0,1 bis 20 kg Pentensäureester I je kg Katalysator und Stunde.

Die Umsetzung kann diskontinuierlich oder kontinuierlich als Festbettreaktion mit Festbettkatalysatoren, beispielsweise im Sumpf- oder Rieselfahrweise, in der Flüssig- oder Gasphase oder als Wirbelbettreaktion mit in auf- und abwirbelnder Bewegung befindlichen Katalysatoren in der Gasphase oder aber in der Flüssigphase mit Homogenkatalysatoren oder suspendierten Festbettkatalysatoren durchgeführt werden.

Ein günstiger Einfluß auf Ausbeute, Selektivität und Katalysator-Standzeit kann weiterhin dadurch erreicht werden, daß die Umsetzung unter Mitverwendung von Verdünnungsmitteln durchgeführt wird. Als Verdünnungsmittel sind z.B. geeignet: Alkohole mit 1 bis 6 Kohlenstoffatomen oder Cyclohexanol, Ether wie Dioxan oder Tetrahydrofuran, chlorierte Kohlenwasserstoffe wie z.B. Methylenchlorid, Chloroform, 1,2-Dichlorethan, aliphatische, cycloaliphatische, aromatischen Kohlenwasserstoffe wie Benzol, Toluol, Cyclohexan, Paraffine. Dabei wendet man je Mol Pentensäureester I 0.2 bis 50, insbesondere 0.5 bis 20 mol Lösungsmittel an. Besonders bevorzugte Verdünnungsmittel stellen Alkohole dar.

Eine mögliche vorteilhafte Ausführungsform des erfindungsgemäßen Verfahrens in der Gasphase sieht z.B. so aus, daß man ein Gemisch aus Pentensäureester I und gegebenfalls Verdünnungsmittel verdampft und dann, zusammen mit Wasserdampf und gegebenenfalls einem Trägergas wie Stickstoff, Kohlendioxid, oder Argon, bei der Gewünschten Reaktionstemperatur gasförmig in eine feste oder eine in auf- und abwirbelnder Bewegung befindliche Katalysatorschicht kondensiert, die organische Phase abgetrennt und anschließend durch fraktionierende Destillation aufgearbeitet. Unumgesetzter Pentensäureester I kann zurückgeführt werden.

Die unter b) verwendeten Katalysatoren sind Sulfonsäuren, z.B. p-Toluolsulfonsäure oder Benzolsulfonsäure, Lewissäuren, wie Bortrifluorid oder Zinkchlorid, nicht-oxidierende Mineralsäuren, wie Schwefelsäure oder Phosphorsäure sowie stark saure Kationenaustauscher, beispielsweise solche aus vernetztem Polystyrol mit Sulfonsäuregruppen oder Phenolharzen mit Sulfonsäuregruppen. Kationenaustauscher, dieser Art sind in HOUBEN-WEYL, Methoden der org. Chemie, BD. I/1, S. 528ff. eingehend beschrieben, worauf hiermit Bezug genommen wird. Unter Lewissäuren werden die als Säuren wirkenden Halogenide der Elemente der III., IV. und V. Hauptgruppe des Periodischen System der Elemente sowie der Nebengruppenelemente verstanden. Dies sind z.B. Aliminiumchlorid, Bortifluorid, Siliciumtetrachlorid, Antimonpentachlorid, Zinntetrachlorid, Zinkchlorid, Titantetrachlorid oder Eisen(III)-chlorid.

Die Umsetzung kann in der Gasphase oder in flüssiger Phase durchgeführt werden. Vorzugsweise wird in flüssiger Phase gearbeitet.

Die einstufige Umsetzung kann diskontinuierlich oder kontinuierlich als Festbettreaktion mit Festbettkatalysatoren, beispielsweise in Sumpf- oder Rieselfahrweise in der Flüssig- oder Gasphase oder aber mit in der Flüssigphase suspendierten oder gelösten Katalysatoren durchgeführt werden.

Die als Katalysatoren verwendeten Sulfonsäuren, Lewissäuren und/oder nicht-oxidierenden Mineralsäuren werden nur in katalytischen Mengen benötigt, z.B.von 0,01 bis 0,25, insbesondere 0,05 bis 0,15, Mol je Mol Pentensäureester. Die stark sauren Kationenaustauscher werden bei der chargenweisen Durchführung allgemein in Mengen von 0,1 bis 40, insbesondere 1 bis 20 Gew.%, bezogen auf den eingesetzten Pentensäureester verwendet. Bei der kontinuierlichen Durchführung wird vorteilhaft eine Belastung von 0,1 bis 10 g Pentensäuremethylester pro g stark sauren Kationenaustauscher gewählt.

Die bevorzugte Umsetzung in der Flüssigphase wird beispielsweise so durchgeführt, daß man ein Gemisch aus dem Ester und Wasser in Gegenwart eines suspendierten oder gelösten Katalysators erhitzt und den gebildeten Alkohol kontinuierlich abdestilliert. Besonders vorteilhaft wird die Hauptmenge des Wasser hierbei erst in dem Maße zu der Reaktionsmischung gegeben, wie der Alkohol abdestilliert. Nach Ablauf der notwendigen Reaktionszeit wird das Reaktionsgemisch zur Gewinnung des 5-Methylbutyrolactons destilliert. Unumgesetzter Ester wird als Vorlauf erhalten und kann zurückgeführt werden.

In einer weiteren, in vielen Fällen besonders vorteilhaften Variante des erfindungsgemäßen Verfahrens wird eine zweistufige Reaktionsführung gewählt. Hierbei wird

A) der Pentensäureester der Formel I zunächst mit Wasser bei 30 bis 200 °C in Gegenwart eines sauren Kationenaustauschers, gegebenenfalls unter gleichzeitiger Abtrennung des gebildeten Alkohols, zur Pentensäure hydrolysiert (Stufe A) und

B) nach Abtrennung des Ionenaustauschers durch Filtration und gegebenenfalls Isolierung der Pentensäure, z.B. durch Destillation oder Extraktion wird die Pentensäure bei Temperaturen von 50 bis 350 °C in Gegenwart von sauren Mitteln zum 5-Methylbutyrolacton cyclisiert (Stufe B).

Im allgemeinen wendet man bei der Hydrolyse (Stufe A) je Mol Pentensäureester 1 bis 200 Mol, insbesondere 50 bis 150 Mol Wasser an.

Die Hydrolyse wird im allgemeinen bei einer Temperatur von 30 bis 200 °C durchgeführt. Vorteilhaft wendet man Temperaturen von 50 bis 120 °C an und führt die Hydrolyse unter Atmosphärendruck durch. Es ist jedoch auch möglich, schwach verminderten oder erhöhten Druck, z.B. bis zu 20 bar anzuwenden.

Die verwendeten stark sauren Kationenaustauscher sind von der gleichen Art, wie sie für die einstufige Arbeitsweise beschrieben sind.

Die Hydrolyse kann chargenweise oder vorteilhaft kontinuierlich, z.B. in einer Rührkesselkaskade durchgeführt werden. Hierbei ist es zweckmäßig, den bei der Hydrolyse gebildeten Alkohol fortlaufend durch Destillation aus dem Reaktionsgemisch abzutrennen. Bei der chargenweisen Durchführung verwendet man im allgemeinen 1 bis 40, insbesondere 5 bis 30 Gew.%, stark sauren Kationenaustauscher, bezogen auf den eingesetzten Pentensäureester. Bei der kontinuierlichen Durchführung wird vorteilhaft eine Belastung von 0,1 bis 10 g Pentensäuremethylester pro g stark sauren Kationenaustauscher gewählt.

Als Produkt der Hydrolyse wird ein Gemisch der Pentensäure mit dem überschüssigen Wasser erhalten. Aus diesem Gemisch läßt sich die Pentensäure durch Destillation oder Extraktion, z.B. mit Ethern wie Methyl-tert.-butylether in reiner Form gewinnen. Es ist jedoch auch möglich, die nach der Hydrolyse erhaltenen Pentensäuren direkt, d.h. ohne Isolierung, in den wäßrigen Lösungen zu cyclisieren.

Die Cyclisierung (Stufe B) wird bei Temperaturen von 50 °C bis 350 °C, vorzugsweise bei 90 bis 250 °C, ausgeführt. Im allgemeinen führt man die Cyclisierung unter Atmosphärendruck durch; es ist jedoch auch möglich, schwach verminderten oder erhöhten Druck, z.B. bis zu 50 bar, anzuwenden.

Als Katalysatoren für die Cyclisierung kommen die gleichen sauren Katalysatoren wie für die Einstufenreaktion angegeben in Betracht. Der Ringschluß der Pentensäure kann in der Gasphase oder in flüssiger Phase erfolgen. Vorzugsweise führt man die Reaktion in flüssiger Phase durch.

Man kann die Umsetzung diskontinuierlich oder kontinuierlich, als Fettbettreaktion mit Festbettkatalysatoren, beispielsweise in Sumpf- oder Rieselfahrweise in der Flüssig- oder Gasphase oder aber mit in der Flüssigphase suspendierten oder gelösten Katalysatoren ausführen.

Die Sulfonsäuren, Lewissäuren und/oder nicht-oxidierenden Mineralsäuren werden nur in katalytischen Mengen benötigt, z.B. in Mengen von 0,002 bis 0,25, insbesondere 0,005 bis 0,1, Mol je Mol Pentensäureester. Die stark sauren Kationenaustauscher werden bei der chargenweisen Durchführung im allgemeinen in Mengen von 0,1 bis 20, insbesondere 0,5 bis 15 Gew.%, bezogen auf den eingesetzten Pentensäureeser angewandt. Bei der kontinuierlichen Durchführung wird vorteilhaft eine Belastung von 0,1 bis 10 g Pentensäure pro g stark sauren Kationenaustauscher gewählt.

Die bevorzugte Umsetzung in der Flüssigphase wird beispielsweise so durchgeführt, daß man die Pentensäure in Gegenwart eines suspendierten oder gelösten Katalysators erhitzt. Nach Ablauf der notwendigen Reaktionszeit, das ist in der Regel 0.1 bis 1 Stunde, wird das Reaktionsgemisch zur Gewinnung des 5-Methylbutyrolactons destilliert.

Das erfindungsgemäße Verfahren zur Herstellung von 5-Methylbutyrolacton besitzt gegenüber den bekannten Verfahren den Vorteil, daß man das Produkt in einfacher Weise in ausgezeichneter Ausbeute aus leicht zugänglichen Ausgangsmaterialien erhält.

Die Pentensäureester sind nämlich durch Carbonylierung von Butadien in Gegenwart von Alkoholen oder durch Eliminierung von Wasser oder Halogenwasserstoff aus Hydroxy- oder Halogenvaleriansäureestern leicht und in hohen Ausbeuten herstellbar.

Das nach dem erfindungsgemäßen Verfahren erhältliche 5-Methylbutyrolacton ist ein wertvolles Zwischenprodukt; u.a. für die Herstellung von Pyrrolidonen wie 5-Methyl-pyrrolidonen sowie von Pharma- und Pflanzenschutzwirkstoffen.

Das Verfahren nach der Erfindung sei an folgenden Beispielen veranschaulicht:

a) Beispiele für die Verwendung von Zeolithen und Phosphaten als Katalysatoren

Die Reaktion wurde in der Gasphase unter isothermen Bedingungen in einem Rohrreaktor (Wendel, 0,6 cm Innendurchmesser, 90 cm Länge) mindestens 6 Stunden lang durchgeführt. Die gasförmigen Reaktionsausträge wurden in Kühlfallen kondensiert, die Phasen getrennt und gewogen und die Reaktionsprodukte durch übliche Methoden abgetrennt und charakterisiert. Die quantitative Bestimmung der Reaktionsprodukte und der Ausgangsstoffe erfolgte gaschromatographisch.

Die für das erfindungsgemäße Verfahren eingesetzten Katalysatoren sind:

Katalysator A

Ein Aluminosilikatzeolith vom Pentasil-Typ wurde unter hydrothermalen Bedingungen bei autogenem Druck und 150 °C aus 65 g hochdispersem $SiO_2$, 20,3 g $Al_2(SO_4)_3 \times 18\ H_2O$ in 1 kg einer wäßrigen 1,6-Hexandiamin-Lösung (Mischung 50:50 Gew.%) in einem Rührautoklaven hergestellt. Nach Abiltrieren und Auswaschen wurde das kristalline Reaktionsprodukt bei 110 °C/24 h getrocknet und bei 500 °C/24 h calciniert. Dieser Aluminosilikatzeolith enthielt 91,6 Gew.% $SiO_2$ und 4,6 Gew.% $Al_2O_3$. Der Katalysator wurde zu 2-mm-Strängen verformt, bei 110 °C/16 h getrocknet und bei 500 °C/24 h calciniert.

Katalysator B

Kommerziell erhältlicher Na-Y-Zeolith wurde mit wäßriger $(NH_4)_2SO_4$-Lösung nach bekannter Methode ionenausgetauscht und zwar so lange bis der Na-Gehalt bei ca. 0,06 Gew.% lag (nach Trocknung 110 °C/2 h und Calcination bei 570 °C/3 h). Das so erhaltene Pulver wurde mit Verformungshilfsmitteln zu Strängen verformt, bei 110 °C getrocknet und bei 500 °C/16 h calciniert.

Katalysator C

Borphosphat wurde hergestellt, indem man 49 g $H_3BO_3$ mit 117 g $H_3PO_4$ (75 %ig) in einem Kneter zusammengab, überschüssiges Wasser abdampfte und das Umsetzungsprodukt zu 3-mm-Strängen verformte. Diese Stränge wurden bei 100 °C getrocknet und bei 350 °C calciniert. Katalysator G enthält 8,77 Gew.% B und 28,3 Gew.% P.

Beispiel 1

Pro Stunde wurden ca. 10 ml 3-Pentensäuremethylester und 10 ml Wasser verdampft und bei 180 °C über 3.5 g Katalysator A geleitet. Nach Kondensation wurde die organische Phase abgetrennt und gaschromatographisch analysiert. Die Analyse ergab folgende Zusammensetzung:
5-Methylbutyrolacton 24,8 %
4-Pentensäuremethylester 3,5 %
3-Pentensäuremethylester 69,8 %
2-Pentensäuremethylester 1,2 %

Beispiel 2

Pro Stunde wurden ca. 10 ml 3-Pentensäuremethylester und 10 ml Wasser verdampft und bei 180 °C über 3.5 g Katalysator B geleitet. Nach Kondensation wurde die organische Phase abgetrennt und gaschromatographisch analysiert:
5-Methylbutyrolacton 47,4 %
4-Pentensäuremethylester 2,8 %
3-Pentensäuremethylester 46,0 %
2-Pentensäuremethylester 2,1 %

Beispiel 3

Pro Stunde wurden ca. 10 ml 3-Pentensäuremethylester und 10 ml eines Gemisches aus Methanol und Wasser (Molverhältnis 1:1) verdampft und bei 180 °C über 3.5 g Katalysator B geleitet. nach Kondensation wurde die organische Phase abgetrennt und gaschromatographisch analysiert:
5-Methylbutyrolacton 23,3%
4-Pentensäuremethylester 8,1%
3-Pentensäuremethylester 64,6%
2-Pentensäuremethylester 2,7%

Beispiel 4

Pro Stunde wurden ca. 10 ml 3-pentensäuremethylester und 10 ml Wasser verdampft und bei 250°C über 3.5 g Katalysator C geleitet. Nach Kondensation wurde die organische Phase abgetrennt und gaschromatographisch analysiert:

| | |
|---|---|
| 5-Methylbutyrolacton | 22,1% |
| 4-Pentensäuremethylester | 3,7% |
| 3-Pentensäuremethylester | 71,7% |
| 2-Pentensäuremethylester | 1,6% |

b) Beispiele für Säuren als Katalysatoren

Beispiel 5

228 g (2,0 Mol) 4-Pentensäuremethylester, 9 g (0,5 Mol) Wasser und 24 g konz. Schwefelsäure (11,7 Mol-% bez. auf Pentensäureester) wurden in einem 0,5-1-Dreihalskolben mit 50 cm Füllkörperkolonne vorgelegt und auf Siedetemperatur (ca. 120 - 130°C) erhitzt. Danach wurde unter ständigem Abdestillieren des bei der Reaktion gebildeten Methanols das restliche Wasser 927 g; 1,5 Mol) innerhalb von 1 h zugegeben. Nach dem vollständigen Abdestillieren des Methanols wurde der Reaktionsansatz über eine kurze Vigreux-Kolonne destilliert. Dabei wurden 167 g (84 % d. Th.) reines 5-Methylbutyrolacton vom Sdp. 65 - 67/10 mbar erhalten.

Beispiel 6

228 g (2, 0 Mol) 3-Pentensäuremethylester (cis/trans-Isomerengemisch; ca. 70 % 3-trans- und 30 % 3-cis-Pentensäuremethylester), 9 g (0,5 Mol) Wasser und 24 g konz. Schwefelsäure 911, 7 Mol-% bez. auf Pentensäureester) wurden in einem 0,5-1-Dreihalskolben mit 50 cm Füllkörperkolonne vorgelegt und auf Siedetemperatur (ca. 120 - 130°C) erhitzt. Danach wurde unter ständigem Abdestillieren des bei der Reaktion gebildeten Methanols das restliche Wasser (27 g; 1,5 Mol) innerhalb von 3 h zugegeben. Nach dem vollständigen Abdestillieren des Methanols wurde die Mischung noch 1 h bei 200°C Sumpftemperatur gerührt und anschließend der Reaktionsansatz über eine kurze Vigreux-Kolonne destilliert. Dabei wurden 172 g (86 % d. Th.) reines 5-Methylbutyrolacton vom Sdp. 65 - 67°C/10 mbar erhalten.

Beispiel 7

228 g (2,0 Mol) 2-trans-Pentensäuremethylester, 9 g (0,5 Mol) Wasser und 24 g konz. Schwefelsäure (11,7 Mol-% bez. auf Pentensäureester) wurden in einem 0,5-l-Dreihalskolben mit 50 cm Füllkörperkolonne vorgelegt und auf Siedetemperatur (ca. 120 - 130°C) erhitzt. Danach wurde unter ständigem Abdestillieren des bei der Reaktion gebildeten Methanols das restliche Wasser (27 g; 1,5 Mol) innerhalb von 4 h zugegeben. Nach dem vollständigen Abdestillieren des Methanols wurde der Reaktionsansatz noch 6 h bei 200°C Sumpftemperatur gerührt und anschließend über eine kurze Vigreux-Kolonne destilliert. Dabei wurden 162 g (81 % d. Th.) reines 5-Methylbutyrolacton vom Sdp. 65 - 67°C/10 mbar erhalten.

Beispiel 8

228 g (2,0 Mol) 2-cis-Pentensäuremethylester, 9 g (0,5 Mol) Wasser und 24 g konz. Schwefelsäure (11,7 Mol-% bez. auf Pentensäureester) wurden in einem 0,5-l-Dreihalskolben mit 50 cm Füllkörperkolonne vorgelegt und auf Siedetemperatur (ca. 120 - 130°C) erhitzt. Danach wurde unter ständigem Abdestillieren des bei der Reaktion gebildeten Methanols das restliche Wasser (27 g; 1,5 Mol) innerhalb von 1 h zugegeben. Nach dem vollständigen Abdestillieren des Methanols wurde der Reaktionsansatz noch 1 h bei 200°C Sumpftemperatur gerührt und anschließend über eine kurze Vigreux-Kolonne destilliert. Dabei wurden 132 g (66 % d. Th.) reines 5-Methylbutyrolacton vom Sdp. 65 - 67°C/10 mbar erhalten.

Beispiel 9

228 g (2,0 Mol) Pentensäuremethylester (Isomerengemisch; ca. 20 Gew.% 4-, 50 Gew.% 3-trans-, 20 Gew.% 3-cis- und 10 Gew.% 2-trans-Pentensäuremethylester), 9 g (0,5 Mol) Wasser und 24 g konz. Schwefelsäure (11,7 Mol-% bez. auf Pentensäureester) wurden in einem 0,5-l-Dreihalskolben mit 50 cm Füllkörperkolonne vorgelegt und auf Siedetemperatur (ca. 120 -130 °C) erhitzt. Danach wurde unter ständigem Abdestillieren des bei der Reaktion gebildeten Methanols das restliche Wasser (27 g; 1,5 Mol) innerhalb von 1 h zugegeben. Nach dem vollständigen Abdestillieren des Methanols wurde der Reaktionsansatz noch 4 h bei 200 °C Sumpftemperatur gerührt und anschließend über eine kurze Vigreux-Kolonne destilliert. Dabei wurden 176 g (88 % d. Th.) reines 5-Methylbutyrolacton vom Sdp. 65 -67 °C/10 mbar erhalten.

Beispiel 10

114 g (1,0 Mol) 4-pentensäuremethylester und 189 g (10 Mol) Wasser wurden zusammen mit 20 g saurem Ionenaustauscher (DOWEX 50 Wx8, Handelsprodukt der Fa. Aldrich-Chemie, Steinheim) zum Rückfluß erhitzt und das entstehende Methanol fortlaufend abdestilliert. Nach 12 h wurde abgekühlt, der Ionenaustauscher abfiltriert und das Filtrat destilliert. Dabei wurden 96 g (96 % d. Th.) reine 4-Pentensäure vom Sdp. 55 °C/2 mbar erhalten.

50 g (0,5 Mol) der so erhaltenen 4-Pentensäure wurde auf 180 °C erhitzt, mit 0,5 g konz. Schwefelsäure (1 Mol-% bez. auf Pentensäure) versetzt, die Mischung 1 h bei 180 °C gerührt und anschließend destilliert. Dabei wurden 46 g (92 % d. Th) reines 5-Methylbutyrolacton vom Sdp. 65 - 67 °C erhalten.

Beispiel 11

Eine Mischung aus 114 g (1,0 Mol) 3-Pentensäuremethylester (cis/trans-Isomerengemisch; ca. 70 % 3-trans- und 30 % 3-cis-Pentensäuremethylester), 180 g (10 Mol) Wasser und 20 g stark saurer Ionenaustauscher (DOWEX 50 Wx8) wurde zum Rückfluß erhitzt und das entstehende Methanol fortlaufend abdestilliert. Nach 12 h wurde abgekühlt, der Ionensaustauscher abfiltriert und das Filtrat destilliert. Dabei wurden 96 g (96 % d. Th.) reine 3-cis/trans-Pentensäure vom Sdp. 107 -108 °C/50 mbar erhalten.

50 g (0,5 Mol) der so erhaltenen 3-cis/trans-Pentensäure wurden auf 180 °C erhitzt, mit 0,5 g konz. Schwefelsäure (1 Mol-% bez. auf Pentensäure) versetzt, die Mischung 1 h bei 180 °C gerührt und anschließend destilliert. Dabei wurden 46 g (92 % d. Th) reines 5-Methylbutyrolacton vom Sdp. 65 - 67 °C erhalten.

Beispiel 12

Eine Mischung aus 114 g (1,0 Mol) 2-trans-Pentensäuremethylester, 180 g (10 Mol) Wasser und 20 g stark saurer Ionenaustauscher (DOWEX 50 Wx8) wurden zum Rückfluß erhitzt und das entstehende Methanol fortlaufend abdestilliert. Nach 150 h wurde abgekühlt, der Ionenaustauscher abfiltriert und das Filtrat destilliert. Dabei wurden 73 g (73 % d. Th.) reine 2-trans-Pentensäure vom Sdp. 55 °C/2 mbar erhalten.

50 g (0,5 Mol) der so erhaltenen 4-Pentensäure wurde auf 180 °C erhitzt, mit 0,5 g konz. Schwefelsäure (1 Mol-% bez. auf Pentensäure) versetzt, die Mischung 1 h bei 180 °C gerührt und anschließend destilliert. Dabei wurden 46 g (92 % d. Th) reines 5-Methylbutyrolacton vom Sdp. 65 - 67 °C erhalten.

Beispiel 13

Eine Mischung aus 1 500 g (13,1 Mol) 3-Pentensäuremethylester (cis/trans-Isomerengemisch; ca. 70 % 3-trans- und 30 % 3-cis-Pentensäuremethylester),2 960 g (164 Mol) Wasser und 200 g stark saurer Ioneaustauscher (DOWEX 50 Wx8) wurde zum Rückfluß erhitzt und das entstehende Methanol fortlaufend abdestilliert. Nach 20 h wurde abgekühlt, der Ionenaustauscher abfiltriert und das Filtrat mit tert.-Butylmethylether extrahiert. Nach dem Abdampfen des Ethers wurden 1 260 g (96 % d. Th.) reine 3-cis/trans-Pentensäure erhalten.

1 260 g (12,6 Mol) der so gewonnenen 3-cis/trans-Pentensäure wurde auf 180° C erhitzt, mit 12,5 g konz. Schwefelsäure (1 Mol-% bez. auf Pentensäure) versetzt, die Mischung 1 h bei 180° C gerührt und anschließend destilliert. Dabei wurden 2 212 g (96 % d. Th) reines 5-Methylbutyrolacton vom Sdp. 65 -67° C erhalten.

## Ansprüche

1. Verfahren zur Herstellung von 5-Methylbutyrolacton, dadurch gekennzeichnet, daß man Pentensäureester der Formel I

$X\text{-}CO_2R$ (I),

in der X die Reste $CH_2=CH\text{-}CH_2\text{-}CH_2\text{-}$,
$CH_3\text{-}CH=CH\text{-}CH_2\text{-}$ oder
$CH_3\text{-}CH_2\text{-}CH=CH\text{-}$ bedeutet
und R für Alkylreste, Cycloalkylreste, Aralkyl- oder Arylreste steht, oder Gemische dieser Ester bei Temperaturen von 50 bis 350° C mit Wasser gegebenenfalls in Gegenwart eines Verdünnungsmittels

a) an Zeolith- und/oder Phosphat-Katalysatoren umsetzt oder

b) in Gegenwart von 0,01 bis 0,25 Mol, pro Mol Pentensäureester, an Sulfonsäuren, Lewissäuren und/oder nicht-oxidierenden Mineralsäuren oder über 0,1 bis 40 Gew.%, bezogen auf den Pentensäureester, stark saurer Ionenaustauscher als Katalysatoren in einer Stufe umsetzt, oder daß man in einer ersten Stufe den Pentensäureester der Formel I, in der X und R die angegebenen Bedeutungen haben, mit Hilfe eines stark sauren Ionenaustauschers als Katalysator zur Pentensäure der Formel I, in der R Wasserstoff bedeutet, hydrolysiert und die so erhaltene Pentensäure in einer zweiten Stufe in Gegenwart von 0,005 bis 0,1 Mol, pro Mol Pentensäure, an Sulfonsäuren, Lewissäuren oder nicht-oxidierenden Mineralsäuren oder über 0,1 bis 20 Gew.%, bezogen auf die Pentensäure, eines stark sauren Ionenaustauschers bei Temperaturen von 50 bis 350° C cyclisiert.

2. Verfahren nach Anspruch 1, dadurch gekennzeichnet, daß man als Pentensäureester Pentensäurealkylester mit 1 bis 6 Kohlenstoffatomen verwendet.

3. Verfahren nach den Ansprüchen 1 und 2, dadurch gekennzeichnet, daß man 0,2 bis 10, insbesondere 0,5 bis 5 Mol, Wasser pro Mol Pentensäureester einsetzt.

4. Verfahren nach den Ansprüchen 1 bis 3, dadurch gekennzeichnet, daß man als Katalysator Zeolithe des Pentasiltyps verwendet.

5. Verfahren nach den Ansprüchen 1 bis 3, dadurch gekennzeichnet, daß man als Katalysator Aluminiumsilikatzeolithe des Pentasil- oder Faujasittyps verwendet.

6. Verfahren nach den Ansprüchen 1 bis 3, dadurch gekennzeichnet, daß man als Katalysator Phosphate der Elemente Bor, Aluminium, Cer, Zirkonium, Eisen, Strontium oder deren Gemische verwendet.

7. Verfahren nach Ansprüchen 1 und 6, dadurch gekennzeichnet, daß man hydrothermal hergestellte oder durch Fällung hergestellte Phosphate verwendet.

8. Verfahren nach Anspruch 1, dadurch gekennzeichnet, daß man als Katalysator gefälltes Borphosphat verwendet.

9. Verfahren nach den Ansprüchen 1 bis 9, dadurch gekennzeichnet, daß man die Umsetzung in Gegenwart von Alkoholen mit 1 bis 6 Kohlenstoffatomen durchführt.

10. Verfahren nach den Ansprüchen 1 bis 3, dadurch gekennzeichnet, daß man

a) in einer ersten Stufe Pentensäureester bei 30 bis 200° C mit 1 bis 200 Mol Wasser in Gegenwart von 5 bis 30 Gew.%, bezogen auf den Pentensäureester, saurer Kationenaustauscher, gegebenenfalls unter fortlaufender Abtrennung des gebildeten Alkohols, hydrolysiert

b) und in einer zweiten Stufe die Pentensäure nach Abtrennung des Ionenaustauschers, in Gegenwart von 0,005 bis 0,1 Mol, je Mol Pentensäure, an Sulfonsäuren, Lewissäuren oder nichtoxidierenden Mineralsäuren oder über 0,5 bis 15 Gew.%, bezogen auf die Pentensäure, stark saurer Ionenaustauscher bei 90 bis 250° C cyclisiert.